# EUROPEAN PATENT APPLICATION

(11) **EP 3 320 836 A1**
(43) Date of publication of application: **16.05.2018**
(21) Application number: 17169365.8
(22) Date of filing: 04.05.2017
(51) Int. Cl.: A61B 5/024, A61B 5/00, A61B 5/103

(54) **METHOD FOR DETECTING HEART RATE AND HEART RATE MONITORING DEVICE USING THE SAME**

(30) Priority: 10.11.2016 US 201615347785
(71) Applicant: HTC Corporation, Taoyuan City 330 (TW)
(72) Inventor: Kao, Chen-Hua, 231 New Taipei City (TW)
(74) Representative: Murgitroyd & Company

(57) **Abstract**

A method of controlling an emitted light strength for a heart rate monitoring (HRM) device is disclosed. The method includes steps of obtaining a surface condition of a skin of a user; determining an emitted strength of an emitted light according to the surface condition of the skin; and determining the heart rate of the user according to the emitted light with the emitted strength.

## Description

### Background of the Invention

### 1. Field of the Invention

The present invention relates to a method for detecting a heart rate and a heart rate monitoring (HRM) device using the same, and more specifically, to a method for detecting a heart rate according to a surface condition of a skin, and an HRM device using the same.

### 2. Description of the Prior Art

Photoplethysmography (PPG) signal is developed to help people to monitor a peripheral hemal circulatory circumstance or a heart rate of a user/patient. PPG signal may be obtained by a noninvasive heart rate monitoring (HRM) device. As technology grows, the HRM device may be a wearable electronic device such as a smart watch or a smart bracelet.

Specifically, the HRM device may comprise a PPG sensor. The PPG sensor emits an emitted light toward a skin of a user/patient, and a reflected light, reflected from the skin and received by the PPG sensor, is used to generate the PPG signal. In the prior art, the HRM device would gradually and incrementally adjust an emitted strength/brightness of the emitted light unit such that a reflected strength of the reflected light is sufficient for the HRM device to identify the PPG signal and accordingly generate a heart rate signal. However, it is time-consuming for the HRM device of the prior art to adjust the emitted light strength to a proper level. Therefore, it is necessary to improve the prior art.

### Summary of the Invention

It is therefore a primary objective of the present invention to provide a method for detecting a heart rate and an HRM device using the same, capable of adjusting the emitted light strength to the proper level faster, to improve over disadvantages of the prior art.

An embodiment of the present invention discloses a method for detecting a heart rate. The method comprises obtaining a surface condition of a skin of a user; determining an emitted strength of an emitted light according to the surface condition of the skin; and determining the heart rate of the user according to the emitted light with the emitted strength.

An embodiment of the present invention further discloses an HRM device comprising a surface condition determining module, for obtaining a surface condition of a skin of a user; a processing unit; and a storage unit, for storing a program code to instruct the processing unit to perform the following steps determining an emitted strength of an emitted light according to the surface condition of the skin; and determining a heart rate of the user according to the emitted light with the emitted strength.

These and other objectives of the present invention will no doubt become obvious to those of ordinary skill in the art after reading the following detailed description of the preferred embodiment that is illustrated in the various figures and drawings.

### Brief Description of the Drawings

FIG. 1 is a schematic diagram of a heart rate monitoring (HRM) device according to an embodiment of the present invention.
FIG. 2 is a schematic diagram of optical spectrum reflectance rate.
FIG. 3 is a schematic diagram of a process according to an embodiment of the present invention.
FIG. 4 is a schematic diagram of a process according to an embodiment of the present invention.
FIG. 5 is a schematic diagram of a HRM device according to an embodiment of the present invention.
FIG. 6 is a schematic diagram of a process according to an embodiment of the present invention.
FIG. 7 is a schematic diagram of a process according to an embodiment of the present invention.

### Detailed Description

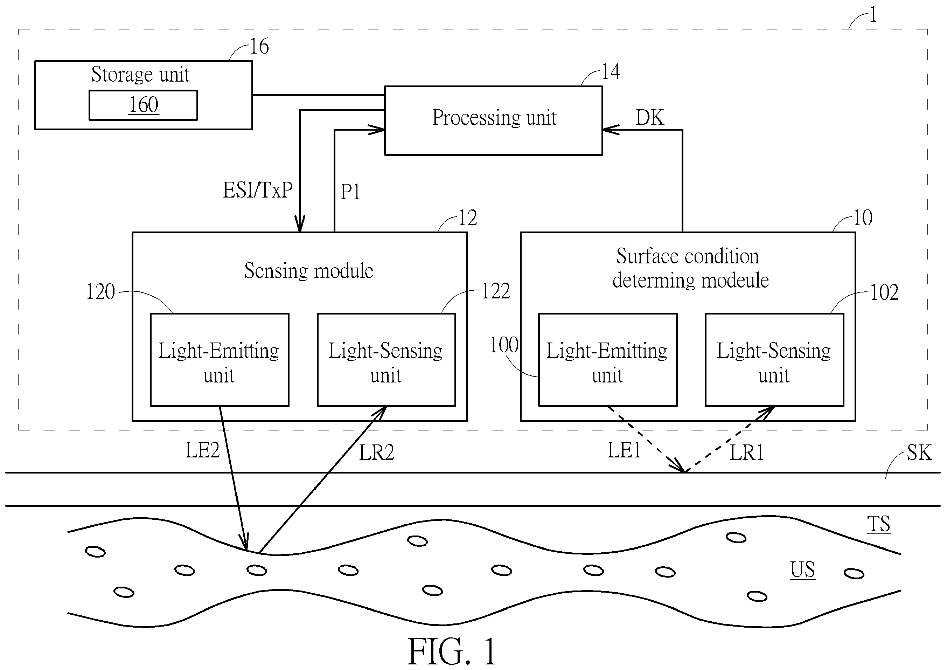

Please refer to FIG. 1, which is a schematic diagram of a heart rate monitoring (HRM) device 1 according to an embodiment of the present invention. The HRM device 1 may be a wearable electronic device such as a smart watch, a smart bracelet, a finger image sphygmomanometer, etc., configured to monitor a hear rate of a user/patient. The HRM device 1 comprises a surface condition determining module 10, a sensing module 12, a processing unit 14 and a storage unit 16. The sensing module 12 may be a photoplethysmogram (PPG) sensor, which is to generate a PPG signal P1. The processing unit 14 may determine the heart rate of the user/patient according to the PPG signal P1. The surface condition determining module 10 is configured to determine a darkness corresponding to a skin of a user, and accordingly generate a darkness result DK. A program code 160, stored in the storage unit 16, is configured to instruct the processing unit 14 to execute steps of a process. The processing unit 14 may be a microprocessor or an application-specific integrated circuit (ASIC). The storage unit 16 may be read-only memory (ROM), random-access memory (RAM), non-volatile memory (e.g., an electrically erasable programmable read only memory (EEPROM) or a flash memory), CD-ROMs, magnetic tapes, floppy disks, optical data storage devices, etc., and not limited herein.

Notably, the sensing module 12 may emit an emitted light with an emitted strength/brightness TxP, where the emitted strength TxP may be in a luminance related measurement. The emitted strength TxP is indicated by the processing unit 14 through an emitted strength information ESI. The processing unit 14 determines the emitted strength information ESI for the sensing module 12 according to the darkness result DK generated by the surface condition determining module 10, such that a reflected strength of a reflected light, corresponding to the emitted light emitted by the sensing module 12, is sufficient for the HRM device 1 to generate the PPG signal P1 and compute a heart rate signal of the user according to the PPG signal P1. Notably, the darkness result DK herein may be regarded as a kind of surface condition of the skin SK of the user.

Specifically, the surface condition determining module 10 may comprise a light-emitting unit 100 and a light-sensing unit 102. The light-emitting unit 100 may emit an emitted light LE1 toward a skin SK, and the first light-sensing unit 102 may receive/capture a reflected light LR1 from the skin SK. The surface condition determining module 10 may determine the darkness corresponding to the skin SK, accordingly generate the darkness result DK, and deliver the darkness result DK. Hence, the processing unit 14 may determine the emitted strength information ESI for the sensing module 12 according to the darkness result DK.

Operations of the surface condition determining module 10 generating the darkness result DK is not limited. In an embodiment, the darkness corresponding to the skin SK is determined based on an optical reflectance rate of the skin SK. Specifically, the surface condition determining module 10 may analyze the reflected light LR1, and obtain an optical spectrum reflectance rate corresponding to the reflected light LR1. According to the reflected optical spectrum reflectance rate, the surface condition determining module 10 may generate the darkness result DK related to the optical spectrum reflectance rate corresponding to the skin SK.

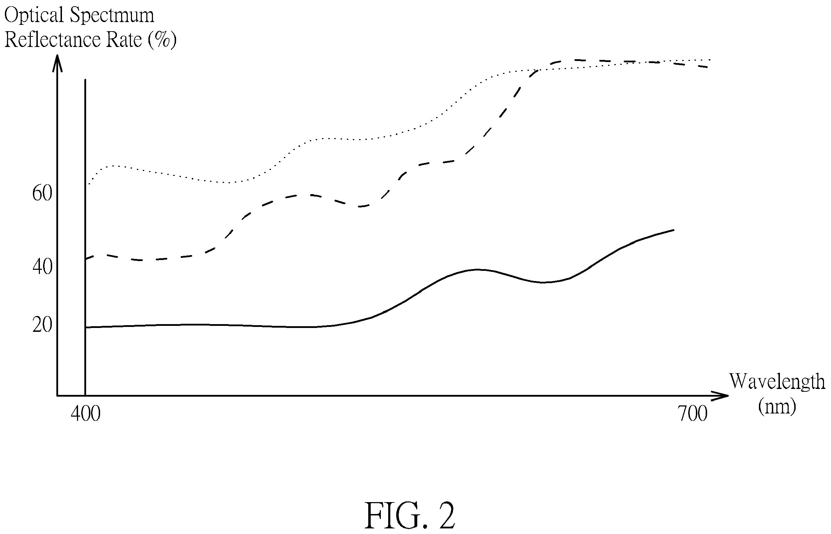

Please refer to FIG. 2, which illustrates curves of optical spectrum reflectance rates corresponding to different skin colors. In FIG. 2, a solid line, a dashed line and a dot line represent curves of the optical spectrum reflectance rates of the reflected light corresponding to a deep skin color, a middle-deep skin color and a light skin color, respectively. As can be seen from FIG. 2, the optical spectrum reflectance rate corresponding to the deep skin color is between 5% and 11%, the optical spectrum reflectance rate corresponding to the mid-deep skin color is between 13% and 38%, and the optical spectrum reflectance rate corresponding to the light skin color is between 29% and 54%.

When the optical spectrum reflectance rate of the reflected light LR1 from the skin SK is lower, it implies that the skin color of the skin SK is deeper/darker (or hair of the user on the skin SK is denser). In such a situation, the sensing module 12 should emit the emitted light with more emitted strength TxP, such that the reflected strength of the reflected light corresponding to the emitted light emitted by the sensing module 12 is sufficient for the HRM device 1 to identify the PPG signal P1, and generate the heart rate correctly.

In an embodiment, the surface condition determining module 10 may compute a statistic of the optical spectrum reflectance rate corresponding to the skin SK, and generate the darkness result DK according to the statistic of the optical spectrum reflectance rate, wherein the statistic may be a mean, a maximum value, a minimum value, etc., of the optical spectrum reflectance rate. In an embodiment, the surface condition determining module 10 may quantize the optical spectrum reflectance rate (or the statistic of the optical spectrum reflectance rate) into one bit or several bits, and generate the darkness result DK based on the quantized optical spectrum reflectance rate (or the quantized statistic of the optical spectrum reflectance rate).

Operations of the processing unit 14 determining the emitted strength information ESI according to the darkness result DK is not limited. In an embodiment, a look-up table (LUT), containing corresponding relationship between the darkness result DK and the emitted strength information ESI, may be stored in the storage unit 16. The processing unit 14 may refer to the look-up table, and output the emitted strength information ESI corresponding to the darkness result DK to the sensing module 12.

The emitted strength information ESI is received by the sensing module 12, which comprises a light-emitting unit 120, a light-sensing unit 122 and a front-end circuit (not illustrated in FIG. 1). The front-end circuit may comprise a digital-to-analog converter (DAC) or an amplifier. The front-end circuit receives the emitted strength information ESI to drive the light-emitting unit 120, such that the light-emitting unit 120 emits an emitted light LE2 with the emitted strength TxP toward the skin SK. Hence, the emitted light LE2 would penetrate through the skin SK, tissues TS, and a capillary vessel US of the user/patient, and a reflected light LR2 corresponding to the emitted light LE2 is reflected from the capillary vessel VS (or from the skin SK). The light-sensing unit 122 is configured to receive/sense the reflected light LR2, and the sensing module 12 generates the PPG signal P1 according to the reflected light LR2, for the HRM device 1 to determine the heart rate signal. Specifically, the reflected light LR2 may be processed by a low-pass filter or a high-pass filter, an amplifier, or an analog-to-digital convertor (ADC), and then the PPG signal P1 is generated accordingly. Further details of the sensing module 12 generating the PPG signal P1 according to the reflected light LR2 is known by the art, which is not narrated herein.

In addition, the sensing module 12 may sense a reflected strength corresponding to the reflected light LR2, and the processing unit 14 may determine the emitted strength information ESI according to the darkness result DK as well as the reflected strength of the reflected light LR2.

In addition, the light-emitting units 100 and 120 may be light emitting diodes (LEDs), and the light-sensing units 102 and 122 may be photodiodes. Preferably, the emitted light LE2 emitted by the light-emitting unit 120 may contain green light, red light or/and infrared ray, and thus the sensing unit 122 is chosen to be sensitive to light with a wavelength corresponding to green light, red light or infrared ray. On the other hand, the emitted light LE1 emitted by the light-emitting unit 100 may have wide optical spectrum bandwidth, and thus the sensing unit 102 is chosen to be sensitive to the reflected light with a wide range of wavelength.

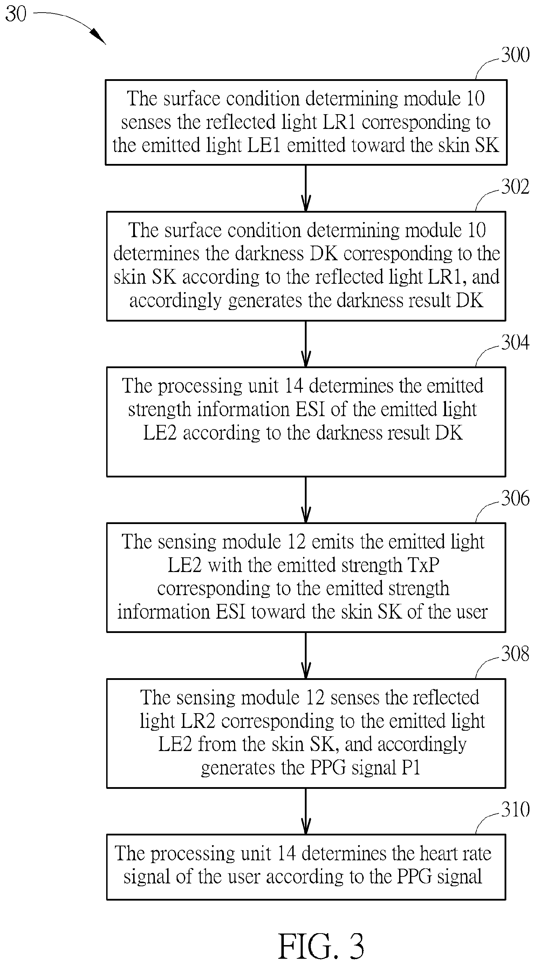

Operations of the HRM device 1 may be summarized as a process 30. As shown in FIG. 3, the process 30 comprises the following steps:
Step 300: The surface condition determining module 10 senses the reflected light LR1 corresponding to the emitted light LE1 emitted toward the skin SK.
Step 302: The surface condition determining module 10 determines the darkness DK corresponding to the skin SK according to the reflected light LR1, and accordingly generates the darkness result DK.
Step 304: The processing unit 14 determines the emitted strength information ESI of the emitted light LE2 according to the darkness result DK.
Step 306: The sensing module 12 emits the emitted light LE2 with the emitted strength TxP corresponding to the emitted strength information ESI toward the skin SK of the user.
Step 308: The sensing module 12 senses the reflected light LR2 corresponding to the emitted light LE2 from the skin SK, and accordingly generates the PPG signal P1.
Step 310: The processing unit 14 determines the heart rate signal of the user according to the PPG signal.

Details of Step 310, in which the processing unit 14 determines the heart rate signal according to the PPG signal, is known by the art, which is not narrated herein. Step 304 and Step 310, executed by the processing unit 14, may be compiled as the program code 160 stored in the storage unit 16. Details of the process 30 may be referred to paragraphs stated in the above, which is not narrated herein.

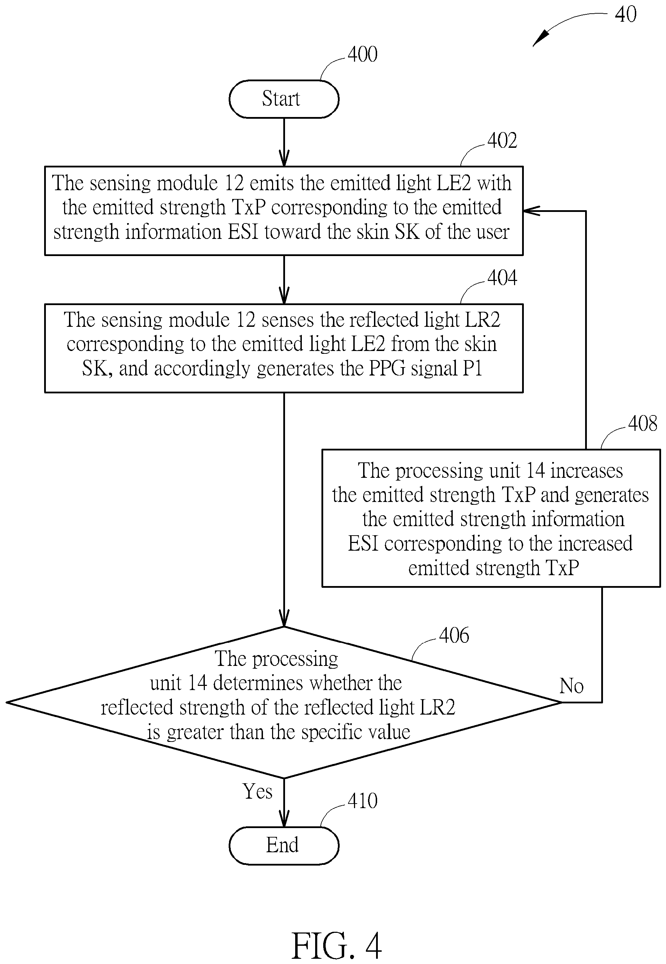

In addition, to adapt to an environment variation (e.g., a variation of environment darkness or brightness), after the emitted light LE2 with the emitted strength TxP is emitted and the reflected light LR2 is received by the sensing module 12, the processing unit 14 may further determine whether the reflected strength of the reflected light LR2 is sufficient, i.e., the processing unit 14 may determine whether the reflected strength of the reflected light LR2 is greater than a specific value. When the reflected strength of the reflected light LR2 is greater than the specific value, there is no need for the HRM device 1 to further increase the emitted strength TxP. Otherwise, when the processing unit 14 determines that the reflected strength of the reflected light LR2 is smaller than the specific value, meaning that the reflected strength of the reflected light LR2 is insufficient for the HRM device 1 to identify the PPG signal P1, the processing unit 14 may further increase the emitted strength TxP. Operations of determining the reflected strength of the reflected light LR2 and adjusting the emitted strength TxP may be performed iteratively, until the reflected strength of the reflected light LR2 is greater than the specific value, which may be summarized as a process 40. As shown in FIG. 4, the process 40 comprises the following steps:
Step 400: Start.
Step 402: The sensing module 12 emits the emitted light LE2 with the emitted strength TxP corresponding to the emitted strength information ESI toward the skin SK of the user.
Step 404: The sensing module 12 senses the reflected light LR2 corresponding to the emitted light LE2 from the skin SK, and accordingly generates the PPG signal P1.
Step 406: The processing unit 14 determines whether the reflected strength of the reflected light LR2 is greater than the specific value.
Step 408: The processing unit 14 increases the emitted strength TxP and generates the emitted strength information ESI corresponding to the increased emitted strength TxP.
Step 410: End.

Details of the process 40 may be referred to the paragraph stated in the above, which is not narrated herein.

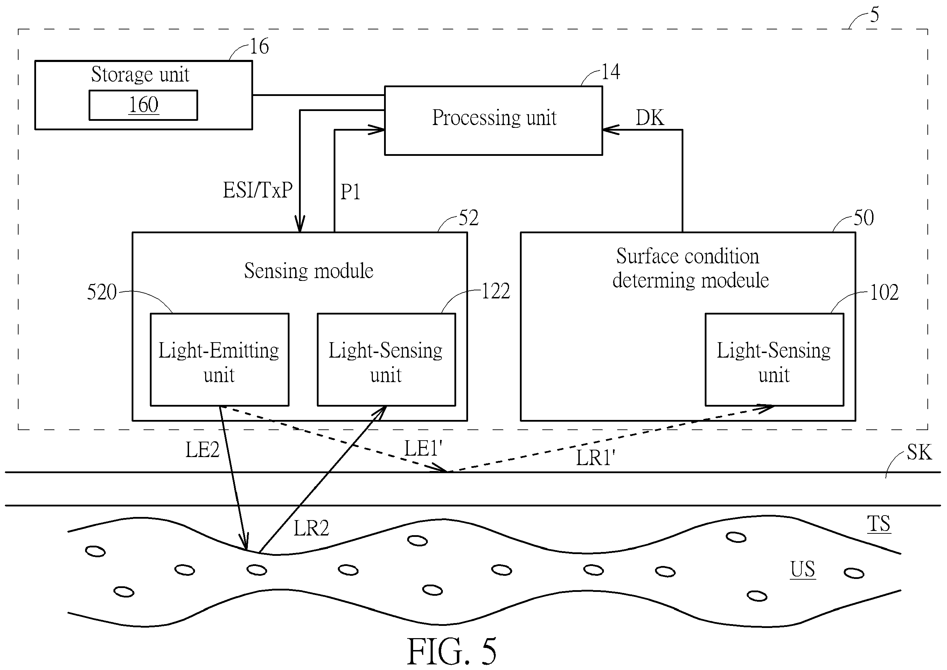

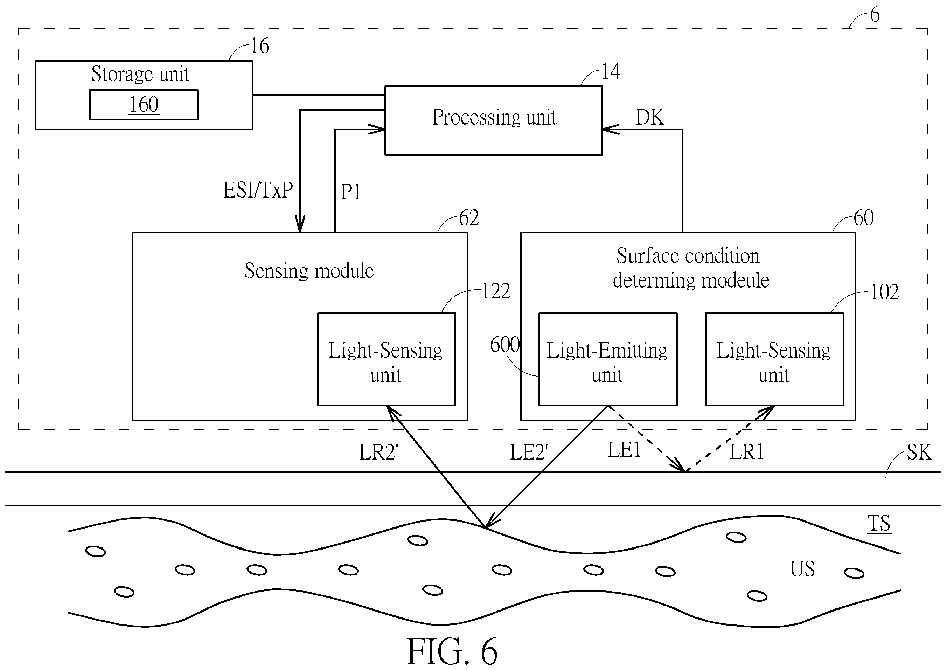

Notably, the embodiments stated in the above are utilized for illustrating the concept of the present invention. Those skilled in the art may make modifications and alterations accordingly, and not limited herein. For example, the HRM device is not limited to comprise two individual light-emitting units 100 and 120. In an embodiment, the light-emitting units 100 and 120 may be integrated as one single light-emitting module. In an embodiment, the HRM device may comprises only one single light-emitting unit. For example, please refer to FIG. 5 and FIG. 6, which are schematic diagrams of an HRM device 5 and an HRM device 6, respectively, according to an embodiment of the present invention. The HRM device 5 and the HRM device 6 are similar to the HRM device 1, and thus, the same components are denoted by the same symbols. Different from the HRM device 1, the HRM device 5 comprises a surface condition determining module 50 and a sensing module 52, where the sensing module 52 comprises a light-emitting unit 520 but the surface condition determining module 50 comprises no light-emitting unit. The light-emitting unit 520 is configured to emit an emitted light LE1' (with wide optical spectrum bandwidth) and the emitted light LE2. A reflected light LR1' corresponding to the emitted light LE1' is received by the light-sensing unit 102. On the other hand, the HRM device 6 comprises a surface condition determining module 60 and a sensing module 62, where the surface condition determining module 60 comprises a light-emitting unit 600 but the sensing module 62 comprises no light-emitting unit. Similarly, the light-emitting unit 600 is configured to emit the emitted light LE1 (with wide optical spectrum bandwidth) and an emitted light LE2'. A reflected light LR2' corresponding to the emitted light LE2' is received by the light-sensing unit 122.

In addition, the surface condition determining module of the present invention is not limited to determine the darkness of the skin SK. In an embodiment, the surface condition determining module of the present invention may be a camera. The camera is configured to capturing an image corresponding to the skin SK of the user, and the process unit 14 may perform an image analysis on the image and obtain a brightness of the image, such that the process unit 14 may determine/adjust the emitted strength TxP according to the brightness of the image. Specifically, the process unit 14 may convert the image into a grey-level picture, and performing a summation over a plurality of grey-level values corresponding to a plurality of pixels within the grey-level picture. The summation result of the plurality of grey-level values would represent the brightness of the image. Note that, the brightness of the image may be regarded as a kind of surface condition of the skin SK of the user. Note that, the brightness of the image may be regarded as a kind of surface condition of the skin SK of the user.

In an embodiment, the surface condition determining module of the present invention may be a color sensor. The color sensor is configured to determine a skin color of the skin SK according to the reflected light. Note that, the skin color may be regarded as a kind of surface condition of the skin SK of the user.

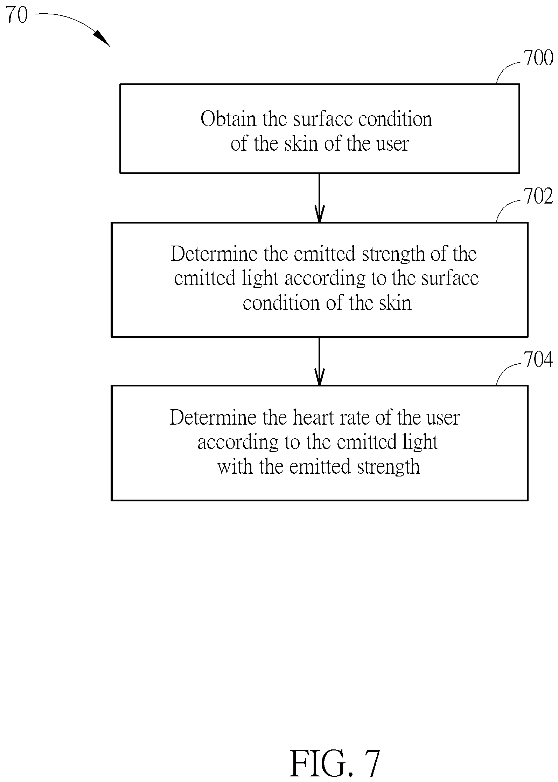

Operations of the HRM device of the present invention may be summarized as a process 70. As shown in FIG. 7, the process 70 comprises the following steps:
Step 700: Obtain the surface condition of the skin of the user.
Step 702: Determine the emitted strength of the emitted light according to the surface condition of the skin.
Step 704: Determine the heart rate of the user according to the emitted light with the emitted strength.

Details of the process 70 may be referred to the paragraph stated in the above, which is not narrated herein.

Note that in the context of this disclosure, a machine readable storage medium or a non-transitory computer-readable medium store programs for use by or in connection with a data processing system, apparatus, or device. In this regard, one example, among others, is a machine readable storage medium embodying a program executable in a data processing system such as the HRM device 1 in FIG. 1. In accordance with such examples, the program may be executed cause the data processing system to perform the process 70. In addition, the machine readable medium may include storage mediums such as read only memory (ROM), random access memory (RAM), magnetic disk storage media, optical storage media, flash memory devices, and may include transmission mediums such as electrical, optical, acoustical, or other form of propagated signals (e.g., carrier waves, infrared signals, digital signals, etc.), etc.

In summary, the present invention utilizes the surface condition determining module to determine the darkness corresponding to the skin of the user, such that the processing unit may determine the emitted light strength according to the darkness corresponding to the skin. Compared to the prior art, the present invention would adjust the emitted light strength to the proper level faster.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. A method for detecting a heart rate, comprising:
obtaining a surface condition of a skin of a user;
determining an emitted strength of an emitted light according to the surface condition of the skin; and
determining the heart rate of the user according to the emitted light with the emitted strength toward the skin.

2. The method of claim 1, wherein the step of obtaining the surface condition of the skin of the user further comprises:
sensing a first reflected light corresponding to a first emitted light toward the skin; and
determining a darkness corresponding to the skin according to the first reflected light;
wherein the surface condition is the darkness corresponding to the skin.

3. The method of claim 2, wherein the step of determining the darkness corresponding to the skin of the user further comprises:
obtaining a reflectance rate corresponding to the first reflected light from the skin; and
determining the darkness corresponding to the skin according to the reflectance rate.

4. The method of claim 1, wherein the step of determining the heart rate of the user according to the emitted light with the emitted strength further comprises:
emitting the emitted light with the emitted strength toward the skin;
sensing a second reflected light corresponding to the emitted light; and
determining the heart rate of the user according to the second reflected light.

5. The method of claim 4, further comprising:
obtaining a reflected strength corresponding to the second reflected light; and
determining the emitted strength of the second emitted light according to the darkness and the received strength corresponding to the second reflected light.

6. The method of claim 5, further comprising:
determining whether the reflected strength corresponding to the second reflected light is greater than a specific value; and
increasing the emitted strength of the emitted light when the reflected strength corresponding to the second reflected light is greater than the specific value.

7. The method of claim 1, wherein the step of obtaining the surface condition of the skin of the user further comprises:
capturing an image of the skin of the user;
performing an image analysis on the image and obtaining a brightness of the image; and
obtaining the surface condition of the skin according to the brightness of the image.

8. The method of claim 7, wherein the step of performing the image analysis on the image and obtaining the brightness of the image further comprises:
converting the image into a grey-level picture;
performing a summation over a plurality of grey-level values corresponding to a plurality of pixels within the grey-level picture; and
obtaining the brightness of the image according to a summation result of the plurality of grey-level values.

9. A heart rate monitoring, HRM, device, comprising:
a surface condition determining module (10), for obtaining a surface condition of a skin of a user;
a storage unit (16), for storing instructions; and
a processing unit (14), connected to the storage unit (16), wherein the storage unit (16) stores, and the processing unit (14) is configured to execute, the instructions of:
determining an emitted strength of an emitted light according to the surface condition of the skin; and
determining a heart rate of the user according to the emitted light with the emitted strength toward the skin.

10. The HRM device of claim 9, wherein the surface condition determining module (10) is configured to sense a first reflected light from the skin and to determine a darkness corresponding to the skin according to the first reflected light, and the surface condition is the darkness corresponding to the skin.

11. The HRM device of claim 10, wherein the surface condition determining module (10) comprises:
a first light-sensing unit (102), for sensing the first reflected light; and
a first light-emitting unit (100), for emitting a first emitted light toward the skin;
wherein the first reflected light is corresponding to the first emitted light.

12. The HRM device of claim 10, wherein the surface condition determining module (10) for determining the darkness corresponding to the skin according to the first reflected light, is further configured to execute instructions of:
obtaining a reflectance rate corresponding to the first reflected light from the skin; and
determining the darkness corresponding to the skin according to the reflectance rate.

13. The HRM device of claim 9, further comprising a sensing module (12), wherein the sensing module (12) comprises:
a second light-sensing unit (122), for sensing a second reflected light corresponding to the emitted light; and
a second light-emitting unit (120), for emitting the emitted light.

14. The HRM device of claim 13, wherein, for determining the heart rate of the user according to the second emitted light with the emitted strength toward the skin, the storage unit (16) stores, and the processing unit (14) is configured to execute, the further instructions of:
determining the heart rate of the user according to the second reflected light from the skin.

15. The HRM device of claim 13, wherein the storage unit (16) stores, and the processing unit (14) is configured to execute, the further instructions of:
obtaining a reflected strength corresponding to the second reflected light; and
determining the emitted strength of the second emitted light according to the darkness and the received strength corresponding to the second reflected light.
